# EUROPEAN PATENT APPLICATION

(11) **EP 4 686 471 A1**
(43) Date of publication of application: **04.02.2026**
(21) Application number: 24382860.5
(22) Date of filing: 01.08.2024
(51) Int. Cl.: A61K 31/137, A61P 35/00, A61P 35/04

(54) **COMPOUND FOR USE AS INHIBITOR OF FASCIN1 IN CANCER**

(71) Applicant: FUNDACION UNIVERSITARIA SAN ANTONIO (UCAM), 30107 Guadalupe (Murcia) (ES); Fundación para la Formación e Investigación Sanitarias (FFIS), 30202 Cartagena (ES); Universidad de Granada, 18071 Granada (ES)
(72) Inventor: RODRIGUEZ MARTINEZ, ALEJANDRO, 30107 GUADALUPE (MURCIA) (ES); GIRALDO RUIZ, LUCIA, 18071 GRANADA (ES); LUQUE FERNANDEZ, IRENE, 18071 GRANADA (ES); DACRUZ RIVEIRO, DIOGO RAFAEL, CARTAGENA (PT); POSTIGO CORRALES, FATIMA, 30107 GUADALUPE (MURCIA) (ES); ALBURQUERQUE GONZALEZ, BEGOÑA, 30107 GUADALUPE (MURCIA) (ES); MONTORO GARCIA, SILVIA, 30107 GUADALUPE (MURCIA) (ES); ARROYO RODRIGUEZ, ANA BELEN, 30202 CARTAGENA (ES); CONESA ZAMORA, PABLO, 30202 CARTAGENA (ES); HURTADO LOPEZ, ANA MARIA, 30107 GUADALUPE (MURCIA) (ES); LUENGO GIL, GINES, 30202 CARTAGENA (ES); PEREZ SANCHEZ, HORACIO, 30107 GUADALUPE (MURCIA) (ES)
(74) Representative: Díaz Pacheco, Maria Desamparados

(57) **Abstract**

The present invention relates to a chemical compound for use in the treatment of cancer by inhibiting fascin 1. It also relates to a pharmaceutical composition comprising said inhibitor of fascin1.

## Description

### Field of the invention

The present invention relates to the field of medicinal products, particularly to therapeutic products against cancer.

### State of the art prior to the invention

Cancer remains one of the most formidable challenges in the medical field, presenting a myriad of complex mechanisms that evade current therapeutic strategies and demand novel approaches to treatment1. The primary cause of death from cancer is attributed to tumor metastasis. The ability of cancer cells to migrate and invade is a hallmark of their metastatic capacity, necessitating the reorganization of the actin cytoskeleton. This reorganization facilitates the creation of protrusions such as filopodia, lamellipodia, and invadopodia, which are instrumental in cancer cell movement. A pivotal protein in this process is Fascin1, which bundles actin filaments (F-actin) and is integral to developing these protrusive structures. Unlike most normal epithelial tissues where it is minimally expressed or not at all, Fascin1 is notably increased in various human cancers. This elevation is linked to enhanced tumor growth, invasion, and metastasis. Fascin1 has been identified as a promising therapeutic target and marker for aggressive cancers. Previous studies have shown its overexpression in different types of cancer like the serrated adenocarcinoma (SAC), a colorectal carcinoma subtype recognized by the WHO for its poor prognosis and more aggressive invasion, as evidenced by increased tumor budding, E-cadherin loss, and higher rates of KRAS or BRAF mutations compared to typical colorectal cancers.

Migrastatin and its derivatives, which effectively inhibit Fascin1, have shown potential in reducing tumor cell movement, invasion, and subsequent metastasis. However, the synthesis of these compounds is complicated by their complex structure, prompting the exploration of other anti-Fascin1 agents, including those derived from indazol-furan-carboxamides. Other molecules, such as the G2 compound and derivates from its chemical structure, have also demonstrated a strong response inhibiting the fascin function. Also, clear progress has been made in the drug repurposing field, obtaining some FDA-Approved compounds:

ES2919724A1 discloses the use of Raltegravir as a fascin1 inhibitor in the treatment of colorectal cancer. However, raltegravir is structurally unrelated to the compound identified in the present invention.

EP3628330A1 discloses the use of Imipramine as a fascin1 inhibitor. However, Imipramine is structurally unrelated to the compound identified in the present invention.

The scientific article Han S, Huang J, Liu B, et al. Improving fascin inhibitors to block tumor cell migration and metastasis. Mol Oncol. 2016;10(7):966-980. doi:10.1016/j.molonc.2016.03.006 discloses the compound G2 as a fascin inhibitor. However, the compound of the present invention is more effective than G2.

In the present invention, we conducted a computer-based screening of the Enamine HTS chemical library (more than 1.360.000 compounds), followed by in vitro studies with the best potential hit to assess the effectiveness of said compounds aiming to halt cancer cell progression

### Description of the invention

As used herein "treatment" refers to administration of a composition, agent or inhibitor to a patient who has a disorder with the purpose to cure, alleviate, relieve, remedy, delay the onset of, prevent, or ameliorate the disorder, the symptom of a disorder, the disease state secondary to the disorder, or the predisposition toward the disorder.

An "effective amount" or "therapeutically effective amount" refers to an amount of the compound or agent that is capable of producing a medically desirable result in a treated patient. The treatment method can be performed in vivo or ex vivo, alone or in conjunction with other drugs or therapy. A therapeutically effective amount can be administered in one or more administrations, applications or dosages and is not intended to be limited to a particular formulation or administration route. An effective amount can be the amount of compound that inhibits fascin 1 activity by at least 50%

The term "subject" or "patient" is not particularly limited, as long as it is an animal. Preferably the subject is a mammalian animal, such as a human, dog, cat, horse, cattle or cow, or any of other types of mammalian animals raised, for example, at zoos, more preferably a human.

The term "colon cancer" as used herein refers to any cancer that originates in the colon or rectum, especially serrated adenocarcinoma, but also gastrointestinal stromal tumor (gist), squamous cell carcinoma, neuroendocrine and sarcoma of the colon.

The terms "mRNA," refers to a polymer of ribonucleotides, messenger Ribonucleic Acid.

The term "inhibit" means to reduce or decrease in activity or expression. This can be a complete inhibition of activity or expression, or a partial inhibition. Inhibition can be compared to a control or to a standard level. Inhibition can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64,65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100%.

The present invention relates to a fascin1 inhibitor for use as a medicament, the fascin 1 inhibitor being Z1362873773, having the following formula:

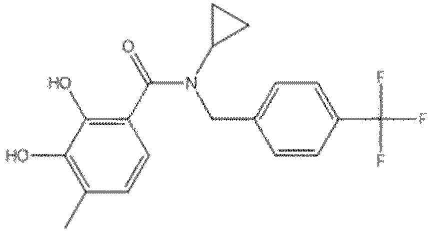

In a particular embodiment, the invention relates to a fascin1 inhibitor for use in the treatment of cancer in a subject.

Z1362873773 is the compound named N-Cyclopropyl-2,3-dihydroxy-4-methyl-N-[[4-trifluoromethyl)phenyl]methyl]benzamide, of chemical formula of C₁₉H₁₈F₃NO₃. In addition, Z1362873773 also includes a pharmaceutically acceptable salt, solvent, isomer and/or ester thereof.

In the present specification, Z1362873773 is also termed "Z13". The PubChem CID of said compound is 75377489

Fascin1 function is closely related to cell motility as it is a cytoskeleton protein, it is important in embrynoic development as cells migrate and differentiate. Hence, It is widely expressed in tissues from ectoderm or endoderm origin.

In another particular embodiment the cancer in that fascin1 is detected is a cancer of ectoderm or endoderm origin.

In another particular embodiment the cancer in that fascin1 is detected is selected form colorectal cancer, triple-negative breast carcinoma and non-small cell lung cancer, preferably colorectal cancer.

In another particular embodiment, the colorectal cancer is serrated adenocarcinoma.

In another particular embodiment, the tumor cells of the serrated adenocarcinoma have wild type KRAS and BRAF genes.

In another particular embodiment, the tumor cells of the serrated adenocarcinoma have at least one mutation in KRAS and/or BRAF genes.A physician would recognize the particular point mutations in either KRAS and BRAF that are associanted with cancer, as an example in the case of KRAS the most relevant point mutations in the polypeptide sequence are: G12C, G12V, G12S, G12D, G12A, G12R and G13R. In the case of BRAF, the most relevant point mutation in the polypeptide sequence is: V600E

In a preferred embodiment the treatment of cancer is suppression of metastatic colonization.

The suppression of metastatic colonization comprises the suppression of metastatic colonization in at least an organ such as lung, liver, and lymph nodes.

Fascin1 stands for Fascin Actin-Bundling Protein 1, the human DNA sequence of Fascin 1 gene can be found in the Ensembl database, code ENSG00000075618. The humanprotein sequence of fascin 1 can be found in the UniprotKB database, code Q16658.

In the present specification the compound NP-G2-044 is a fascin1 inhbitor with a No. CAS: 1807454-59-6

In the present specification the compound NP-G2-029 is a fascin1 inhbitor disclosed in Han S, Huang J, Liu B, et al. Improving fascin inhibitors to block tumor cell migration and metastasis. Mol Oncol. 2016;10(7):966-980. doi:10.1016/j.molonc.2016.03.006

In a particular embodiment, the cancer is a cancer in which fascin1 is detected in at least 10% of the cancer cells in a sample obtained from the patient. In an additional embodiment fascin1 expression level is detected in at least 15% of cancer cells form a sample obtained from the patient, preferably at least 20%, more preferably, at least 25%, even more preferably at least 30%, even more preferably at least 40%, even more preferably at least 50%, even more preferably at least 60% and even more preferably at least 70%.

The description provides a method for determining whether a subject has, or is at risk of having, cancer in that fascin 1 is detected in at least 10% of cancer cells. The method includes (i) obtaining from the patient a sample, preferably a biopsy of the cancer; (ii) measuring in the sample the amount of cancer cells positive for fascin1 protein by immunostaining (for example, immunohistochemistry or immunofluorescence), (iii) calculate the % of cancer cells positive for fascin1 from the total cancer cells of the sample.

The subject is determined to have, or to be at risk of having, cancer if the cells positive for fascin1 protein expression are at least 10% of the total cancer cells within the sample obtained from the patient. The sample can be a body fluid sample or a solid biopsy sample. The method can also be used for determining whether a subject has, or is at risk of having, recurrence of metastatic cancer, or for determining whether a subject has, or is at risk of having, cancer or metastatic cancer that is resistant to chemotherapeutics or targeted therapies. More specifically, the subject is determined to have, or be at risk of having recurrence of metastatic cancer, or cancer or metastatic cancer that is resistant to chemotherapeutics or targeted therapies, if the cells positive for fascin1 protein expression are at least 10% of the total cancer cells within the sample obtained from the patient

In a particular embodiment, the methodology for assessing fascin1 positive cells is by immunohistochemical staining as described elsewhere (Conesa-Zamora et al. 2013): To consider a tumour cell to be positive for fascin1 expression a diffuse cytoplasmic staining of fascin1 in cancer cells is required.

Fascin1 positive cancer cells can be detected by polymerase chain reaction, Enzyme Linked Immuno Sorbent Assay or immunostaining, immunohistochemistry or immunofluorescence. A skilled person would know how to identify fascin1 positive cancer cells using regular protocols and materials.

In a particular embodiment, fascin1 is detected by immunostaining, preferably immunohistochemistry.

In another particular embodiment the immunohistochemistry protocol and materials employed, such as fascin1 antibody are those described in Conesa-Zamora et al. 2013 particularly supplementary information S1.

When a skilled person or a pathologist is analysing a patient sample stained for fascin1 he might find that staining is not uniform within the sample or the cancer cells of the sample a immunohistochemically expression or staining of fascin1 in vascular endothelium is considered an internal positive control whereas the absence of expression in normal mucosa as a negative.

A pathologist or skilled person in the field would know how to perform the staining procedure. In addition, the skilled person would know how to differentiate cancer cell from other cells within the patient sample. For example, by a regular Hematoxilin and Eosin staining (H&E) or by using specific tumor cells marker such as cytoqueratins: CK20, CK7, CKAE1 or CKAE3.

In another particular embodiment, the level of fascin 1 is detected by qPCR or Next generation sequencing as mRNA.

Z13 acts an inhibitor of fascin1 by binding to it and preventing cancer cell invasion and metastasis.

In a particular embodiment, the method of treatment comprises administering to a patient an effective amount of Z13.

The particular dose administered according to this invention will o be determined by the particular circumstances surrounding the case, including the Z13 administered, the exact route of administration, the particular cancer being treated, the recipient subject, and similar considerations.

An expert in the field, a physician, would know the best route of administration, dosage and concentration to achieve a therapeutical effect.

Z13 can be administered parenterally, orally, nasally, or rectally. The term "parenteral" as used herein refers to subcutaneous, intracutaneous, intravenous, intrmuscular, intraarticular, intraarterial, intrasynovial, intrasternal, intrathecal, intralesional, or intracranial injection, as well as any suitable infusion technique.

In a particular embodiment, the concentration of Z13 administered to a subject is between 0.01 µM and 1000 µM, or 0.1 µM and 1000 µM, preferably 1 µM and 100 µM, more preferably 5 µM and 50 µM, or 10 µM and 40 µM or 15 µM and 30 µM.

In another particular embodiment, the amount of Z13 administered to a subject can be an amount from as low of about 0.1 µg/ml, about 0.2 µg/ml, or about 0.5 µg/ml, to a high of about 100 µg/ml, about 200 µg/ml, or about 500 µg/ml. For example, the amount of Z13 can be from about 0.1 µg/ml to about 100 µg/ml, from about 0.2 µg/ml to about 200 µg/ml, from about 0.5 µg/ml to about 500 µg/ml, from about 0.1 µg/ml to about 10 µg/ml, from about 0.1 µg/ml to about 20 µg/ml, from about 0.1 µg/ml to about 50 µg/ml, from about 0.5 µg/ml to about 20 µg/ml, from about 0.5 µg/ml to about 50, from about 0.5 µg/ml to about 100 µg/ml, from about 0.5 to about 200 µg/ml, from about 0.5 µg/ml to about 500 µg/ml, from about 15 µg/ml to about 25 µg/ml, from about 15 µg/ml to about 50, from about 18 µg/ml to about 30 µg/ml, from about 18 µg/ml to about 50 µg/ml.

In other embodiments, the dosage can be an amount to result in a concentration in one or more body fluids of a subject of from a low of about 0.1 µg/ml, about 0.2 µg/ml, or about 0.5 µg/ml, to a high of about 100 µg/ml, about 200 µg/ml, or about 500 µg/ml. For example, the dosage can be an amount to result in a concentration in one or more body fluids of a subject of from about 0.1 µg/ml to about 100 µg/ml, from about 0.2 µg/ml to about 200 µg/ml, from about 0.5 µg/ml to about 500 µg/ml, from about 0.1 µg/ml to about 10 µg/ml, from about 0.1 µg/ml to about 20µg/ml, from about 0.1 µg/ml to about 50 µg/ml, from about 0.5 µg/ml to about 20 µg/ml, from about 0.5 µg/ml to about 50, from about 0.5 µg/ml to about 100 µg/ml, from about 0.5 to about 200 µg/ml, from about 0.5 µg/ml to about 500 µg/ml, from about 15 µg/ml to about 25 µg/ml, from about 15 µg/ml to about 50, from about 18 µg/ml to about 30 µg/ml, from about 18 µg/ml to about 50 µg/ml.

To confirm the inhibition of fascin1 or treatment, one can evaluate and/or verify the inhibition of cancer cell survival, hypoxic survival, metastatic survival, or metastatic colonization using technology known in the art before and/or after the administering step. Exemplary technologies include CT-scans or PET-scans of organs of the body.

An additional object of the invention relates to a pharmaceutical composition comprising the fascin1 inhibitor described above, that comprises at least a pharmaceutically acceptable carrier.

A "pharmaceutically acceptable carrier," after administered to or upon a subject, does not cause undesirable physiological effects. The carrier in the pharmaceutical composition must be "acceptable" also in the sense that it is compatible with the active ingredient and can be capable of stabilizing it. One or more solubilizing agents can be utilized as pharmaceutical carriers for delivery of an active compound. Examples of a pharmaceutically acceptable carrier include, but are not limited to, biocompatible vehicles, adjuvants, additives, and diluents to achieve a composition usable as a dosage form. Examples of other carriers include colloidal silicon oxide, magnesium stearate, cellulose, sodium lauryl sulfate, and D&C Yellow 10.

The pharmaceutical composition of the invention can be administered parenterally, orally, nasally, or rectally. The term "parenteral" as used herein refers to subcutaneous, intracutaneous, intravenous, intramuscular, intraarticular, intraarterial, intrasynovial, intrasternal, intrathecal, intralesional, or intracranial injection, as well as any suitable infusion technique.

The pharmaceutical composition of the invention can be administered as a sterile injectable which can be a solution or suspension in a non-toxic parenterally acceptable diluent or solvent. Such solutions include, but are not limited to, 1,3-butanediol, mannitol, water, Ringer's solution, and isotonic sodium chloride solution. In addition, fixed oils are conventionally employed as a solvent or suspending medium (e.g., synthetic mono- or diglycerides). Fatty acid, such as, oleic acid and its glyceride derivatives, are useful in the preparation of injectables, as are natural pharmaceutically acceptable oils, such asolive oil or castor oil, polyoxyethylated versions thereof. These oil solutions or suspensions also can contain a long chain alcohol diluent or dispersant such as, carboxymethyl cellulose, or similar dispersing agents. Other commonly used surfactants, such as, Tweens or Spans or other similar emulsifying agents or bioavailability enhancers, which are commonly used in the manufacture of pharmaceutically acceptable solid, liquid, or other dosage forms also can be used for the purpose of formulation.

A composition for oral administration can be any orally acceptable dosage form including capsules, tablets, emulsions and aqueous suspensions, dispersions, and solutions. In the case of tablets, commonly used carriers include, but are not limited to, lactose and corn starch. Lubricating agents, such as, magnesium stearate, also are typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

In a particular embodiment the pharmaceutical composition for oral administration has at least one of the pharmaceutically acceptable carrier disclosed in EP2529741B1

Pharmaceutical compositions for topical administration according to the described invention can be formulated as solutions, ointments, creams, suspensions, lotions, powders, pastes, gels, sprays, aerosols, or oils. Alternatively, topical formulations can be in the form of patches or dressings impregnated with active ingredient(s), which can optionally comprise one or more excipients or diluents. In some preferred embodiments, the topical formulations include a material that would enhance absorption or penetration of the active agent(s) through the skin or other affected areas.

A therapeutic agent can be administered in vivo or ex vivo, alone or in a composition together with other drugs or therapy, i.e., a cocktail therapy. For example, in the treatment of tumors, particularly malignant tumors, the agents can be used alone or in combination with, e.g., chemotherapeutic, radiotherapeutic, apoptopic, anti-angiogenic agents and/or immunotoxins or coaguligands. In some embodiments, the administration of a pharmaceutical composition of two or more agents/therapies is concurrent. In other embodiments, a first agent/therapy is administered prior to a second agent/therapy. Those of skill in the art understand that the formulations and/or routes of administration of the various agents/therapies used may vary.

In a particular embodiment the pharmaceutical composition includes administering to the person an additional therapeutic agent. Examples of the additional therapeutic agent include one or more selected from the group consisting of 5 -fluoro uracil, Oxalip latin, Irinotecan, Capecitabine, Gemcitabine, Cetuximab, Taxol, Avastin, folinic acid (leucovorin), Regorafenib, Zaltrap, topoisomerase I inhibitors, NKTR-102, Tivantinib, PX-866, Sorafenib, Linifanib, kinase inhibitors, Telatinib, XL281 (BMS-908662), Robatumumab and IGF1-R inhibitors.

The pharmaceutical composition of the invention can be administered as a subsequent treatment or simultaneously with other therapies.

In another particular embodiment, the pharmaceutical composition can be administered with a variable administration interval, as an example from 1 to 7 times per week, preferably from 2 to 6 times per week, more preferably from 3 to 5 times per week.

In another particular embodiment, the effective dose of Z13 and the pharmaceutical composition that comprises it depends on the nature of the conditions, the way of execution and the pharmaceutical formulation, and will be defined by the experience of a physician through the application of the usual investigations for a dose increase. The effective dose of Z13 can be considered to be from 0.0001 to approximately 1000 mg/kg body weight per day, or from 0.01 to approximately 500 mg/kg body weight per day, or from 0.01 to 100 mg/kg body weight per day, or from 0.05 to approximately 10 mg/kg body weight per day. For example, the daily dose for an adult person of approximately 70 kg body weight will be in the range of 1 mg to 1000 mg, or between 5 mg and 500 mg, and can be done by single or multiple doses, daily or not.

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Brief description of drawings

**Figure 1.** (a) 2D representation of the pharmacophore model for the G2 compound generated by LigandScout (b) 3D representation of the pharmacophore model for the G2 compound generated by LigandScout. HBA: hydrogen bond acceptor, HBD: hydrogen bond donor, H: hydrophobic.
**Figure 2****.** Comparison between entre the chemical structure and their pharmacophore model of the G2 compound (a) and the compound obtained for VS from Enamime library (Z1362873773).
**Figure 3****.** Comparison between the tridimensional chemical structure of the G2 compound (light gray) and the Enamine compound obtained by Virtual Screening, Z1362873773 (dark gray).
**Figure 4****.** Denaturation curves of wild-type Fascin in the absence of inhibitor (circle), in the presence of BDP-13176 (square), and in the presence of the pure compounds Z1362873773 (gray).
**Figure 5****.** Actin bundling by Fascin HTS image assay. Top, images of Fascin alone and Fascin in the presence of Actin are shown, as quality controls of the correct function of bundling mediated by fascin. In the medium and bottom, in the presence of the Z1362873773 compound and Fascin, the formation of Actin fibers is reduced to almost none. The images resemble those of Actin alone, indicating that the bundling activity of Fascin is likely specifically inhibited.
**Figure 6****.** Pymol session showing the interactions between the compound Z1362873773 and the crystallographic structure for Fascin (PDB:6B0T) obtained by the docking calculation using the AutoDock Vina and LeadFinder. In bold, those residues match with the critical known residues in the interactions with the fascin structure. Pink interactions show the hydrophobic interactions, the red-colored interactions indicate the hydrogen bonds, green-colored interactions are π-Stacking interactions detected, and cyan blue-colored interactions correspond to the π-cation interactions.
**Figure 7****.** RMSD for protein (light gray color) and ligand (dark gray color) in the NP-G2-029-fascin (A) and Z1362873773-fascin (B) complexes during the Molecular Dynamics (MD) simulation.
**Figure 8****.** MMPBSA analysis of NP-G2-029-fascin (A) and Z1362873773-fascin (B) complexes. The graph shows the van der Waals contribution (VdW Energy, in dark gray color, middle line), electrostatic contribution (Elec. Energy, dark gray color, upper line), and Total Energy (in light gray color, bottom line)
**Figure 9****.** Z13 compound its more effective in affecting the cell viability of patient-derived colon cancer organoids than compound NP-G2-029.

### Examples

With the aim of finding new anti-fascin1 compounds we carried out a funnel-like workflow in which *in silico* compound library screening rendered selected molecules for their potential to bind fascin1 which were subsequently evaluated *in vivo* by a physical chemistry assay for their real interaction with fascin1. All these experiments explained below confirm that Z13 is a compound with fascin1 binding activity and with fascin1-dependent anti-colorrectal cancer effects both in *in vitro* and *in vivo* assays. Since Z13 binds directly to fascin1 and the anti-tumoral effects of raltegravir are fascin1-dependent, we confirm that its therapeutic action is due to this interaction. As fascin1 is overexpressed in other types of cancer also associated with bad prognosis such as triple-negative breast carcinoma and lung cancer, raltegravir will have beneficial effect in other types of tumor in addition to colorectal cancer, such as triple negative breast cancer.

### Materials and methods

### Library generation

First, the HTS compounds library from Enamine (HTS Collection (Enamine, 2024); https://enamine.net/compound-collections/screening-collection/hts-collection.) company was selected to perform the Ligand-Based Virtual Screening calculations. The library contains more than 1.300.000 compounds with a broad chemical space between them, which makes the library highly versatile. This library is much more complex that the FDA approved molecules that only accounts for around 3000 compounds The compound set was split into 217 files to allow the parallelization and optimization of the corresponding calculations. From the command line, we launched the command *idbgen* (available under the LigandScout license) to generate a .Idb format file for each one of the sections in which the library was split.

### Pharmacophore model generation

The next step was to generate a pharmacophore model for the G2 compound, known in the bibliography as an effective fascin inhibitor (Han et al). To obtain the model, we obtained an sdf file with the chemical structure of the inhibitor from PubChem (Kim S., J. Chen, T. Cheng, et al. 2023. PubChem 2023 update. Nucleic Acids Research 51: D1373-D1380. https://doi.org/10.1093/nar/gkac956). The sdf file was uploaded to the LigandScout GUI), and the corresponding pharmacophore model was generated. Those pharmacophore features that do not influence the fascin binding or inhibition were removed. The model was saved in pmz format to screen it against the Enamine library generated in the previous step.

### Ligand-based Virtual Screening by Ligand Scout

A massive Virtual Screening calculation was performed via the in-house software called Metascreener developed by our research group (https://github.com/bio-hpc/metascreener). The pharmacophore model was screened against all the complete Enamine library generated. The maximum number of features to omit (-*a*, *--allow_omit*) for this calculation was 0. The aim was to obtain all the compounds whose pharmacophore features match those of the G2 model. Finally, a post-filter of the results was carried out.

### Biophysical characterization

The selected compounds have been tested through two independent assays based on different principles. Firstly, a primary assay was conducted using Differential Scanning Fluorimetry (Thermofluor), which assesses the compound's ability to interact with the protein, inducing changes in its denaturation temperature. Concurrently, validation was carried out using a F-actin-bundling assay by Fascin.

### Differential Scanning Fluorimetry (DSF) / Thermofluor. Study of Binding to Fascin 1

For this assay, 384-well plates were used, and a final assay volume of 10 µL was employed. With the aid of the Echo^{®} Acoustic Liquid Handling (Labcyte), 400 nL of each compound, from an initial stock of 10 mM and 100% DMSO, was dispensed into four wells to obtain 4 replicates of each, at a final concentration of 400 µM. Two quality control measures for the assay were used: a control of native Fascin, where the protein is present with DMSO at the same concentration as the compounds, and a control for inhibitory activity, in the presence of BDP13176, at a concentration of 400 µM. Each control was placed in 2 complete columns of the plate, constituting 32 repetitions of each condition. The thermal stability profile was measured on the CFX384 Touch Real-Time PCR Detection System (BioRad), and the Tm values in the native protein, as well as the shift in the inhibition control and in each of the compounds, were obtained. The 'Z-Factor' was calculated as an informative parameter of the assay quality.

### F-actin bundling assay

An image-based assay to visualize F-actin bundling mediated by fascin cross-linking was implemented. Labeling was performed with phalloidin conjugated to a commercial fluorescent probe (Alexa Fluor 488-Phalloidin, Thermofisher), directed at the positive charges of the F-actin filaments and bundles. Thus, F-actin structures can be visualized using fluorescence microscopy. The Operetta CLS High Content Analysis System (Perkin Elmer) was used to visualize and capture the images. Subsequently, the images were processed and analyzed using Harmony^{™}.

Each selected compound (300 nL) was dissolved in 100% DMSO from an initial stock (10 mM) using Echo^{®} Acoustic Liquid Handling (Labcyte). Next, an amount of 15 µL of pure fascin at a concentration of 0.5 µM in buffer (20 mM Hepes, 100 mM NaCl, pH 7.45) was dispensed onto a plate in a 384-well format (Corning) using an automatic dispenser (Thermo Multidrop Combi, Fisher), and allowed to incubate for 30 min. Subsequently, 15 µl of polymerized actin was added at a concentration of 0.5 µM (in 100 mM KCI, 20 mM Tris-HCl, pH 7.5, 2 mM MgCl2, 1 mM DTT, 1 mM ATP; Cytoskeleton Inc.). This resulted in a final concentration of 100 µM of the chemical compounds. After another 30 min of incubation, 10 µl of Alexa Fluor-488 Phalloidin (diluted 50-fold from 100% methanol stocks) was added to stain F-actin, and the mixture was incubated in the dark for 1 h. Next, 25 µl of the final solution was transferred to a 384-well plate coated with poly-d-lysine (Corning) and incubated for another 20 min before imaging.

Three quality control assays were performed. Two controls corresponded to negative controls for fascin cross-linking activity. These included F-actin in the absence of fascin to verify that actin bundling depends on the presence of fascin. Fascin at a 1:1 molar ratio was used as a positive control. BDP-13176, a known fascin inhibitor, was used at a concentration equal to that of the compounds tested. In each test, 16 repetitions were performed in different wells of each control.

### Cell culture: Organoids

Patient-derived colorectal cancer organoids were established and cultured using IntestiCult^{™} Organoid Growth Medium (Human) following the manufacturer's protocol (Stemcell Technologies). The medium from each well intended for passage was removed without disturbing the domes; each well was then filled with 500 µl of ice-cold D-PBS, the solution was pipetted up and down to facilitate matrix breakage, and the suspensions were transferred to a 15 ml conical tube, which was repeated at least once to ensure complete retrieval of all organoids, with well checks conducted under an inverted microscope. Subsequently, the tubes were centrifuged at 290g for 5 min at a temperature between 2-8 °C, the supernatant was discarded, without disturbing the organoid pellet, and then treated with 1 ml of TrypLE^{™} Express Solution and incubated for 5 min at 37 C. After incubation, 100 µl of FBS was mixed with the suspension, the tubes were centrifuged at 250 g for 5 minutes at 2-8 °C, and the supernatant was discarded. To the obtained pellet, 1000 µl of DMEM/F-12 + 1% BSA was added and the number of organoids in the suspension was counted using a Countess^{™} 3 Automated Cell Counter with Countess Reusable Slides (Invitrogen, Thermo Fisher Scientific). Considering that the domes of the 96-well plate corresponded to 5 µl and each dome had 5000 cells, the correct amount of suspension to be used for the assay was calculated, always considering the 1:1 ratio of cell suspension and Matrigel^{®} and that each treatment/condition was performed in quadruplicate. Pre-wetting the pipette tips with cold DMEM/F-12 + 1% BSA before manipulating the cell suspension was used to prevent sticking to the wall of the pipette tip, and to place the 96-well plate in the incubator for at least 30 min before starting the assay. After plating, the plates were placed in an incubator at 37 °C and 5% CO2 for 15 min to allow the solidification of the domes. Then, approximately 100 µl of D-PBS was added to the wells in the borders to avoid evaporation of the cell culture medium, and 50 µL of complete IntestiCult^{™} Organoid Growth Medium at room temperature (without Y-27632 and gentamicin) was added. After 48 h, the control with the drug carrier (0.1 % DMSO) and the respective drugs in serial dilutions from 0.1 to 100 µM were added to the corresponding wells (in quadruplicate) at a total volume of 100 µl per well. Subsequently, the plates were left in the incubator for 5 days, and photos of the wells were taken every day using an inverted microscope, as a way to register the growth and the treatment effect on the organoids. On the fifth day, cell viability was determined using the CellTiter-Glo^{®} Luminescent Cell Viability Assay (Promega) following the manufacturer's guidelines. Luminescence was measured using a Spectramax ID3 plate reader (Molecular Devices, VWR).

### Molecular modeling

We conducted molecular modeling for the fascin structure to find the interaction between the predicted compounds and the fascin. First, we used the PDB code 6B0T, corresponding to the binding between the crystallographic structure of the fascin and the NP-G2-029, a derivative of the G2 compound.

The structure was preprocessed using MAESTRO Schorindger software. The chain A was extracted, and the corresponding hydrogens and charges were added to the structure. The new structure was saved in mol2 format. A similar protocol was used with the AutoDockTools¹³ software, doing the same tasks and applying the AD type to the atoms. In this case, the structure was saved in pdbqt format.

Regarding the predicted ligands, they were converted to mol2 and pdbqt format using ChemAxon (MolConvert tools) and AutoDockTools, respectively. The corresponding charges were added in each case.

### Blind Docking

When the protein and the candidate ligand files have been processed, two Blind Docking calculations were performed using the NP-G2-044 Fascin (PDB:6B0T) and the free-protein (PDB:3P53) structures to explore the conformational space and the corresponding possible binding site of the predicted compound. For each Blind Docking, we use two docking software: Lead Finder and AutoDock Vina. A consensus between both methods was carried out to determine the average pose.

### Target Docking

Now, we have a docking targeted in the Actin Binding Site 2 coordinates, where the NP-G2-044 binds to the fascin in the 6B0T pdb structure, and the pose for the best clusters has been found. Lead Finder and AutoDock Vina were used with the respective consensus between both.

### MD simulations

We launched Molecular Dynamics simulations at 100 ns on the same fascin structure 6b0t for the poses obtained by the BD calculations, corresponding to the known binding sites (actin-binding sites 1 and 2, tubulin-binding site) and the one with the best docking score in the blind docking conducted. For this, we first generated the topology of the ligand for each pose using an automatic script that utilizes ACPYPE

We followed the subsequent steps of molecular dynamics simulations using GROMACS 2022.3. These simulations were launched on the Picasso server using GPU (NVIDIA A100-SXM4-40GB) and 4 GB of RAM. We first created the protein topology using the gmx pdb2gmx command, specifying AMBER99SB as the forcefield. We defined the simulation box, solvated, and added ions. The next step was an energy minimization stage of 2000 ps. After that, a single NvT equilibration stage of 50000 ps and 5 NpT equilibration stages of 50000 ps each were carried out. Finally, the dynamics were run at 100 ns, and the final trajectory generated was extracted in different frames to compare the ligands' stability and movement regarding the binding pose.

### Trajectories analysis

The results obtained by MD simulation were analyzed via ASGARD, an in-house tool developed by the group (https://github.com/bio-hpc/ASGARD). The analysis calculated the non-bonded interactions and hydrogen bonds between the final ligand. Also, we study the protein-ligand complex stability and the flexibility and dynamic of the fascin that the ligand has produced with the interaction with the binding site. The needed input files were prepared for the tool, and the plots and raw data were generated.

### Data analysis

Data are expressed as mean ± standard deviation (SD). Data were analyzed for statistical differences by the Student's t-test for paired and unpaired data after testing for normal distribution of the data. For in vitro experiments, one-way analysis of variance (ANOVA) was performed followed by a Tukey poshoc test to compare each group. Differences were considered significant at an error probability of Pp < 0.05. SPSS 18.0 software was used for the rest of statistical analyses (SPSS, Inc, Chicago, Illinois, USA)

### Results

### Ligand-based in silico screening

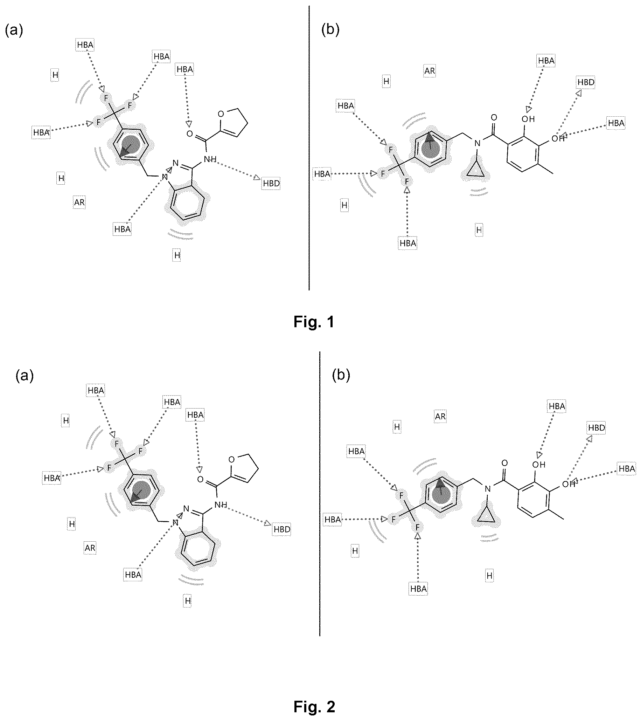

We obtained 212 compounds with all the features matching the pharmacophore model for G2 generated by LigandScout (Figs. 1-3). Those molecules with a Relative-Pharmacophore score higher than 0.97 were chosen for the consequent steps. Lastly, the 13 compounds available in Enamine were tested in vitro in the next step of the study.

### Thermofluor assays validate Z1362873773 binding to fascin1 in vitro

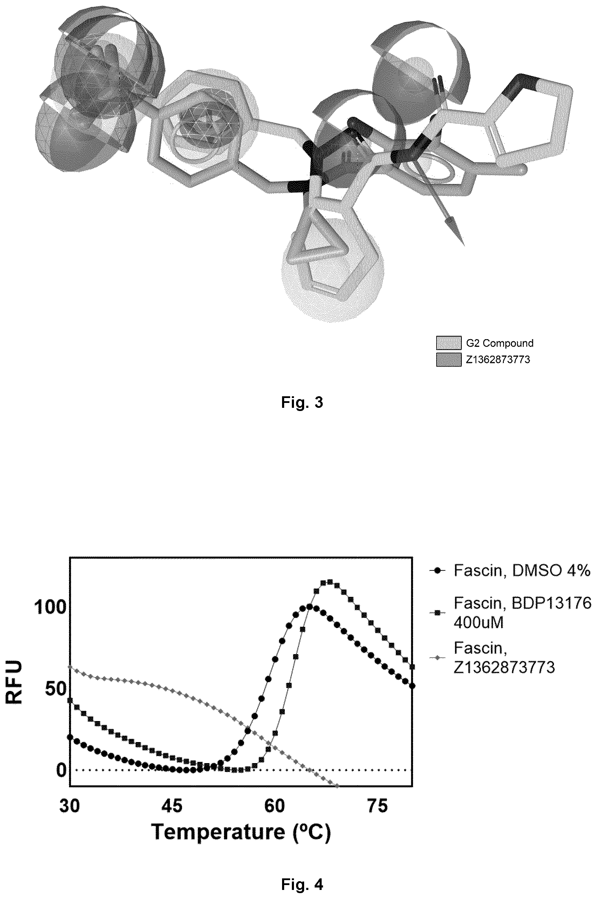

The binding to fascin1 for Z1362873773 was evaluated using differential scanning fluorimetry. The findings reveal that at pH 7.4, fascin1 unfolds through a singular transition without showing any dependency on concentration and exhibits a high level of DMSO tolerance. The average melting temperatures (Tm) for fascin1 without any bound compounds were recorded using different internal filters (FAM, HEX, and T-Red), serving as a baseline to assess Tm changes upon compound binding. Fig. 4 (Tm,FAM = 55.6 ± 0.5 °C, Tm,HEX = 56.0 ± 0.0 °C, Tm,Tred = 56.2 ± 0.6 °C, with error values representing the standard deviation for the seven replicates used as internal controls on each plate).

### Z1362873773 reduces the fascin1 bundling activity

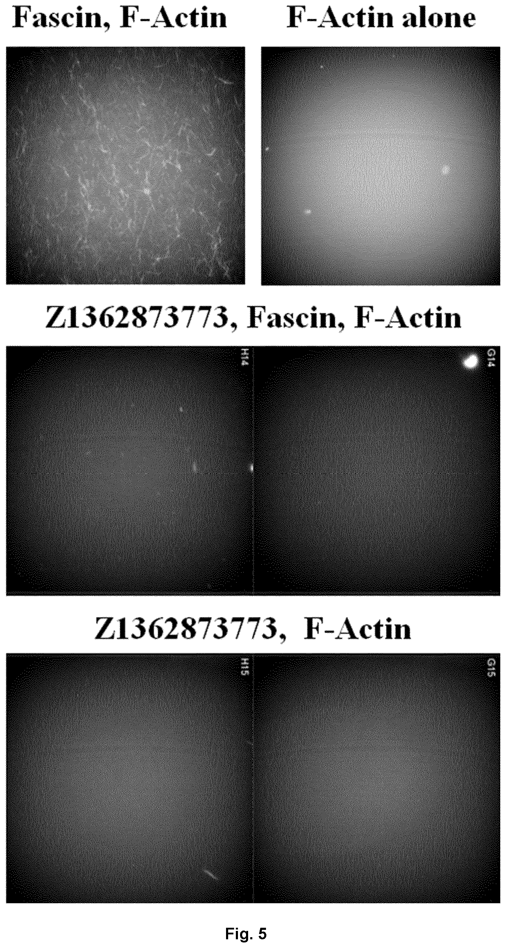

In the presence of the Z1362873773 compound, the formation of Actin fibers is significantly reduced. F-actin bundling assay imaging analyses (Fig 5.) show that the structures formed resemble those of Actin alone, without any signs of the typical bundling associated with Fascin activity. This suggests that the Z1362873773 compound effectively disrupts the interaction between Actin and Fascin, thereby specifically inhibiting the bundling activity of Fascin. The reduction in Actin fiber formation highlights the potential of the Z1362873773 compound as a targeted inhibitor, which could have implications for understanding the mechanisms of cytoskeletal regulation and developing therapeutic strategies against diseases where Fascin-mediated actin bundling plays a critical role.

### Blind Docking finds a top pose in the Actin Binding Site 2

We performed two docking experiments using two different fascin structures. 6B0T corresponds to the structure obtained when NP-G2-029 (G2 analog) binds to actin-binding site 2. Blind Docking using AutoDock showed the pose inside the NP-G2-029 binding site as the first cluster with -8.9 kcal/mol. In the LeadFinder method, Blind Docking calculations were also performed in the free protein structure (PDB:3p53) to explore the structural flexibility of fascin. In the AutoDock Vina results, the poses for the top three and four clusters were found close to the position of actin-binding site 2, and the third cluster pose interacted with Phe216, one of the key residues for actin-binding site 2. In the results obtained from LeadFinder, the first and second clusters were located at actin-binding site 2, exhibiting binding energies of 8.54 and -7.39 kcal/mol, respectively. Notably, cluster 1 also interacted with Phe216 through a hydrogen bond. Regarding the Blind Docking calculations performed for the 6b0t structure, using AutoDock Vina and LeadFinder methods, actin-binding site 2 was detected in the top 1 cluster. The binding energy values were 8.92 and 8.72 kcal/mol for AutoDock Vina and Lead Finder, respectively. Furthermore, the key residues in the binding site were identified in both cases. We found hydrogen bond interactions between the compound and Phe216 and other hydrophobic interactions with crucial residues such as Ile93, Trp101, and Phe14.

### Target Docking shows several interactions with Actin Binding Site 2 key residues

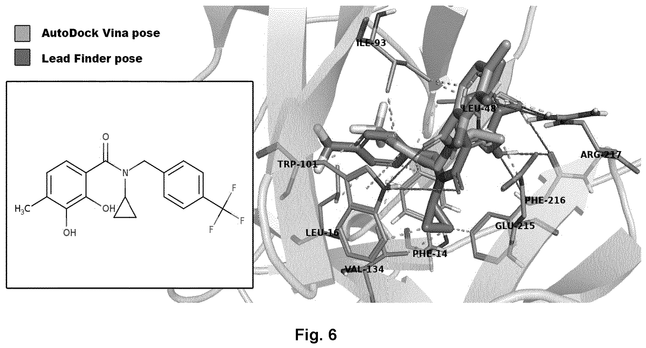

The docking scores obtained by the AutoDock Vina and LeadFinder techniques were -8.35 and -8.27 kcal/mol, respectively. After that, we obtained the specific interactions found by each method (Table 1). The interactions between the compound and the specific fascin residues in the binding site 1 found by AutoDock Vina calculations were the following: Phe14 (3.53 Å), Leu48 (3.80 Å), Ile93 (3.66 Å, 3.30 Å) and Glu215 (3.48 Å) for hydrophobic interactions; Trp101 (2.81 Å) and Arg217 (3.94 Å) for hydrogen bonds; and Trp101 (4.76 Å) for π-Stacking. Regarding the interactioSSns obtained by LeadFinder software, we found hydrophobic interactions between the ligand and the residues Phe14 (3.82 Å), Leu16 (3.37 Å), !!e93 (3.33 Å, 3.27 Å), Trp101 (3.15 Å, 3.39 Å), Val134 (3.67 Å, 3.58 Å) y Phe216 (3.06 Å). LeadFinder also detects hydrogen bonds interaction with Trp101 (4.06 Å), Phe216 (3.00 Å, 3.04 Å, 2.81 Å) y Arg217 (3.41 Å). Fig. 6. The comparison between AutoDock Vina and LeadFinder is shown in Table 1

**Table 1. Interactions between Z1362873773 and the fascin residues detected by AutoDock Vina and Lead Finder calculations.**

| **Software** | **Docking Score (kcal/mol)** | **Hydrophobic Interactions (Å)** | **Hydrogen Bonds (Å)** | **π-Stacking Interactions (Å)** | **π-Cation Interactio ns (Å)** |
|---|---|---|---|---|---|
| AutoDock Vina | -8.35 | **Phe14** (3.53), **Leu48** (3.80), **Ile93** (3.66, 3.30), Glu215 (3.48) | **Trp101** (2.81), Arg217 (3.94) | **Trp101** (4.76) | Arg217 (4.86) |
| LeadFinder | -8.27 | **Phe14** (3.82), **Leu16** (3.37), Ile93 (3.33, 3.27), **Trp101** (3.15, 3.39), **Val134** (3.67, 3.58), **Phe216** (3.06) | **Trp101** (4.06), **Phe216** (3.00, 3.04, 2.81), Arg217 (3.41) | - | - |

### MD analysis shows the Z1362873773 stability in the Actin Binding Site 2 pocket

We performed an MD simulation of Z1362873773 to study the stability of the ligand in the complex over time and in an aqueous system, considering fascin flexibility. In addition, we ran another MD simulation for the NP-G2-029 Fascin complex as a comparative test with a known fascin inhibitor bound to the actin-binding site 2 system.

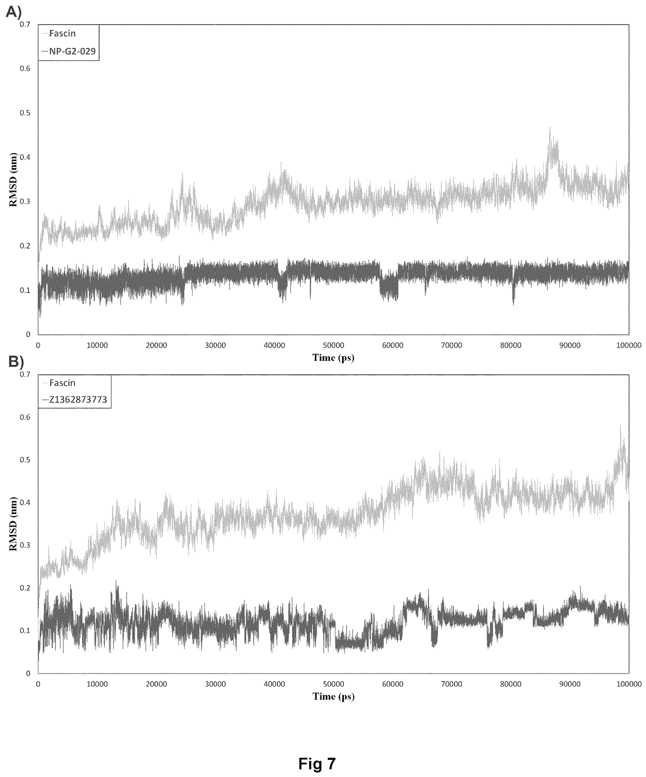

The initial focus was on the RMSD during the simulation for the protein as the ligand, a key indicator of the system's stability in both cases. Fig. 7 illustrates the stabilization of the ligands in each complex, with both NP-G2-029 and Z1362873773 RMSD values maintaining a consistent range of 0.1 to 0.2 throughout the MD. The fascin structure, a known flexible protein, demonstrated significant flexibility during the simulation time, as expected.

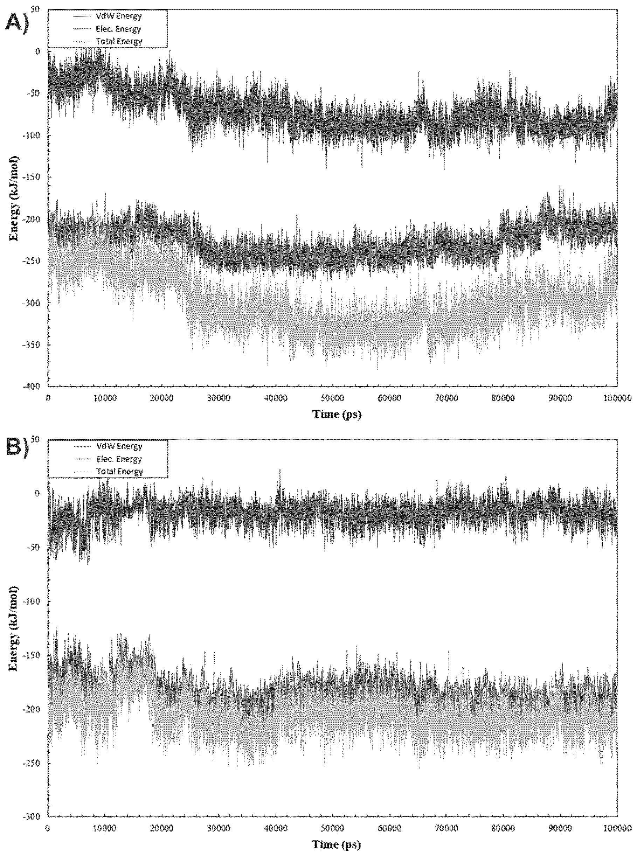

Molecular Mechanics Poisson-Boltzmann Surface Area (MMPBSA) analysis estimates the binding free energy between a protein and its ligand. We performed MMPBSA calculations for both complexes to examine the stability and strength of the binding between NP-G2-029 and Z1362873773 in actin-binding site 1 of fascin. The NP-G2-029 fascin complex stabilized the binding energy from the first 20 ns to between -250 and -300 kJ/mol (Fig. 8A). Regarding the Z1362873773 compound, the binding energy of its complex with fascin started to stabilize in the first 10 ns, remaining at binding energy values of -200 and -250 -kJ/mol2 (Fig. 8B). These analyses also demonstrate that the van der Waals contributions are more prominent than electrostatic interactions.

Finally, we determined the main non-bonded interactions obtained by MD simulations. At this point, most of them coincided with the docking calculation results. Two more residues were highlighted for the NP-G2-029 Fascin complex than the rest. Phe216 and Asp217 reveal high values of Total Energy, obtaining -29.1 and 23.5 kJ/mol for Van der Waals energy and -9.3 and -11.1 kJ/mol for electrostatic energy, respectively. Regarding Z73, the interactions obtained followed a more even pattern. Several residues report total energy values between -10 and -20 kJ/mol. Among them, we can see some of them that match with the docking results and are known as crucial residues in the fascin union, such as Phe14 (E_total= -13.3 kJ/mo!2), Leu48 (E_total= -10.1 kJ/mo!2), Trp101 (E_total= -17.9 kJ/mo!2), Val134 (-11.9 kJ/mol2) and Phe216 (-15.7 kJ/mol2).

Consequently, the MD simulations correlated with the experimental results, showing Z1362873773 as a molecule binding to actin-binding site 1. The consensus of the RMSD, free binding energy validation by MMPBSA, and interaction analysis present a potential ligand with a stable and strong union with fascin, avoiding its function.

### Effect of Z1362873773 in Patient-derived colorectal cancer organoids

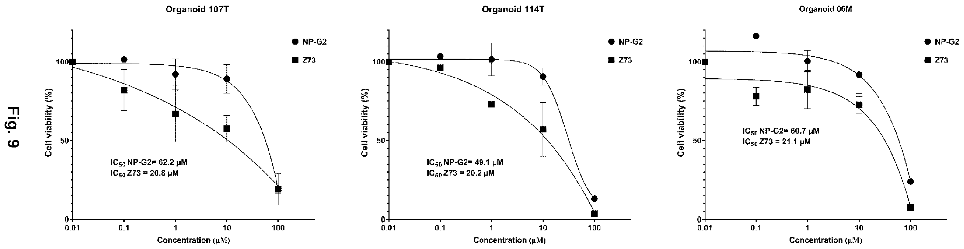

The effect on cell viability of Z1362873773 was studied on patient-derived colorectal cancer organoids, NP-G2-029 was used as a control. In three independent samples, Z13 was shown to be more effective than the control, as the IC50 value was lower in all cases. Fig. 9. These results demonstrate that the compound of the invention has an advantage than the compound of the state of the art

## Claims

1. A fascin1 inhibitor for use as a medicament, the fascin 1 inhibitor being Z1362873773, having the following formula:

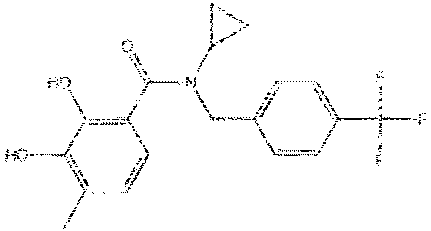

2. The fascin1 inhibitor according to the preceding claim for use in the treatment of cancer in a subject.

3. The fascin1 inhibitor for use, according to any of the preceding claims, wherein the cancer has an ectoderm or endoderm origin.

4. The fascin1 inhibitor for use, according to any of the preceding claims, wherein the cancer is selected from colorectal cancer, triple-negative breast carcinoma and non-small cell lung cancer, preferably colorectal cancer.

5. The fascin1 inhibitor for use, according to the preceding claim, wherein colorectal cancer is serrated adenocarcinoma.

6. The fascin1 inhibitor for use, according to any of the preceding claims, wherein the treatment of cancer is suppression of metastatic colonization.

7. A pharmaceutical composition comprising the fascin 1 inhibitor described in any of the preceding claims 1 to 6, that comprises at least a pharmaceutically acceptable carrier for use as a medicament.

8. The pharmaceutical composition for use, according to the preceding claim, that is administered to a patient parenterally, orally, nasally, or rectally.

9. The pharmaceutical composition for use, according to any of the above claims 7 to 8, wherein the effective dose of Z1362873773 is from 0.0001 to approximately 1000 mg/kg body weight per day.
